# EUROPÄISCHE PATENTANMELDUNG

(11) **EP 1 475 057 A1**
(43) Veröffentlichungstag der Anmeldung: **10.11.2004**
(21) Anmeldenummer: 04006175.6
(22) Anmeldetag: 19.03.2004
(51) Int. Cl.: A61F 2/36

(54) **Gelenkkugel**

(30) Priorität: 14.04.2003 DE 10318374
(71) Anmelder: ESKA Implants GmbH & Co., 23556 Lübeck (DE)
(72) Erfinder: Grundei, Hans, Dr., 23558 Lübeck (DE)
(74) Vertreter: Fuchs Mehler Weiss & Fritzsche

(57) **Zusammenfassung**

Es wird eine Gelenkkugel (1) für ein künstliches Hüftgelenk beschrieben, die dazu geeignet ist, in einer künstlichen Hüftgelenkspfanne eine Rotations- und Schwenkbewegung auszuführen. In die Kugeloberfläche sind wenigstens drei zirkulär um die Polachse (P) verlaufende Nuten (2) ohne Verbindungskanäle untereinander eingebracht.

## Beschreibung

Die vorliegende Erfindung betrifft eine Gelenkkugel für ein künstliches Hüftgelenk, die dazu geeignet ist, in einer künstlichen Hüftgelenkspfanne eine Rotations- und Schwenkbewegung auszuführen.

Derartige Gelenkkugeln sind seit langem bekannt. Sie bestehen üblicherweise aus Metall oder einem Keramik(verbund)werkstoff. Die künstliche Hüftgelenkspfanne weist üblicherweise ein Inlay auf, welches aus beispielsweise Polyethylen, aus einem Keramik(verbund)werkstoff oder aber aus Metall bestehen kann.

Ein grundsätzliches Problem hierbei ist die Reibung zwischen den Gelenkteilen, die ihrerseits zu Verschleißerscheinungen führt, die medizinische Komplikationen nach sich ziehen können.

Als Beispiel für ein künstliches Hüftgelenk, bei dem eine gattungsgemäße Gelenkkugel zum Einsatz kommt, sei ein solches mit einer metallischen Beckenpfanne genannt, die in das Acetabulum gesetzt wird und die ein Inlay aus Polyethylen aufweist. Die Gelenkkugel besteht in diesem Falle aus Keramik oder Metall. Bei diesen Materialpaarungen ist es eine regelmäßige Erfahrung, dass Patienten Osteolysen als Resultat von Polyethylenabrieb entwickeln.

Ein viel versprechender Ansatz, die Reibung zwischen den Gleitpartnern zu minimieren (low friction) und somit einer Verschleißminimierung Vorschub zu leisten, ist bekannt aus der EP 1 030 626 B1. Der darin beschriebene Ansatz geht davon aus, flächenhafte Paarungen von Gleitpartnern für künstliche Gelenkimplantate so weiterzubilden, dass der Flüssigkeitsfilm der Synovia im Spalt zwischen den Gelenkteilen mit hoher Wahrscheinlichkeit nicht abreißt, so dass die Synovia ihre Funktion als Gelenkschmierflüssigkeit zuverlässig ausüben kann. In der Druckschrift wird vorgeschlagen, dass neben dem Spalt für die Synovia zwischen den Gleitpartnern die Oberfläche zumindest eines Gleitpartners regelmäßig angeordnete Vertiefungen aufweist, die nicht zusammenhängend, also ohne Verbindungskanäle untereinander, ausgeführt sind. Die Vertiefungen in der Oberfläche eines Gleitpartners üben eine Sammelfunktion für die Synovia aus, die ihrerseits einen puffernden Effekt hin zum anderen Gleitpartner ausübt. Hierfür sind die Vertiefungen im Querschnitt kalottenförmig ausgebildet. Sie bilden ein genügend großes Reservoir für die Synovia und bieten die Möglichkeit einer guten Umspülung und Austausch der in den Vertiefungen gespeicherten Synovia gegen nachströmende Synovia. So ist stets dafür Sorge getragen, dass die Gleitpartner nicht direkt aufeinander abrollen oder aufeinander gleiten, sondern stets ein Film von Synovia zwischen ihnen die Reibung reduziert. Weniger Abriebspartikel sind die unmittelbare Folge, wodurch die Langzeitstabilität des Implantates noch verbessert wird.

Die Herstellung der Gleitpartner gemäß dem Stand der Technik ist enorm aufwendig. Namentlich das Einbringen der kalottenförmigen Vertiefungen in die Oberfläche ist eine Präzisionsarbeit und erfordert entsprechende Maschinen und Zeitaufwand.

Demgegenüber ist es die Aufgabe der vorliegenden Erfindung, eine gattungsgemäße Gelenkkugel so weiterzubilden, dass einerseits eine Reibungsminimierung und damit eine Verschleißminimierung erreicht wird bei erheblich reduzierten Herstellungskosten.

Gelöst wird diese Aufgabe dadurch, dass wenigstens drei zirkulär um die Polachse der Gelenkkugel verlaufende Nuten ohne Verbindungskanäle untereinander in die Kugeloberfläche eingebracht sind.

Durch die erfindungsgemäße Ausgestaltung wird zunächst eine Kontaktflächenminimierung erzeugt, da im Bereich der Nuten kein direkter Kontakt zwischen der Gelenkkugel und der künstlichen Hüftgelenkspfanne besteht. Die zirkulär verlaufenden Nuten werden nach Implantation gefüllt sein mit Synovia, aber keinen abpuffernden Effekt wie die Vertiefungen im Falle des beschriebenen Standes der Technik, sondern vielmehr eine tragende Rolle ausüben. Darüber hinaus entsteht durch die flüssigkeitsgefüllten Nuten ein Vakuum zwischen der Gelenkkugel und der Hüftgelenkspfanne, welches seinerseits ein deutliches Luxationshemmnis darstellt. Die Gelenkkugel ist relativ preisgünstig herzustellen, da die Nuten in der Gelenkoberfläche beispielsweise durch Präzisionsfräsen erzeugt werden können. Der Aufwand gegenüber dem Gleitpartner gemäß dem Stand der Technik ist jedenfalls deutlich reduziert, wodurch sich auch die Herstellungskosten reduzieren.

Gemäß einer vorteilhaften Ausführungsform ist vorgesehen, dass die Nuten eine Tiefe von bis zu 2 mm aufweisen. Dies erlaubt die Wirkung der Nuten als ausreichendes Reservoir für die Synovia, um eben die erwähnte tragende Funktion auszuüben, aber auch um ein ausreichendes Vakuum zu erzeugen, welches ein zusätzliches Luxationshemmnis darstellt. Eine besonders optimierte Tiefe der Nuten stellt sich bei 1 mm ein.

Gemäß einer noch weiteren vorteilhaften Ausführungsform ist vorgesehen, dass die Nuten einen gerundeten Querschnitt aufweisen.

Dieser gerundete Querschnitt begünstigt eine für die tragende Funktion besonders günstige Druckverteilung.

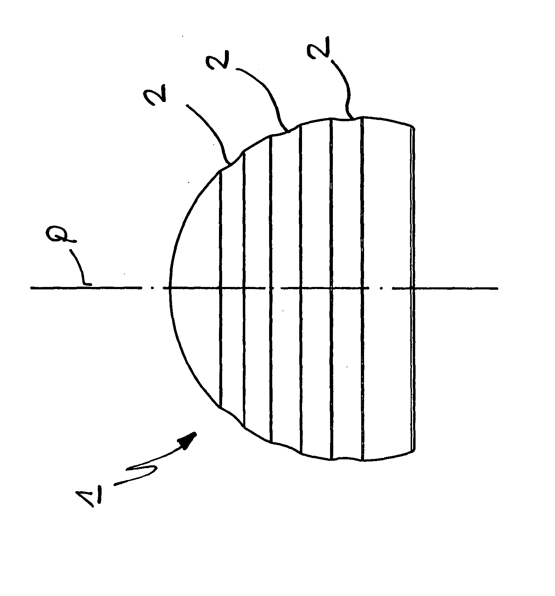

Die Erfindung wird anhand der einzigen Zeichnungsfigur näher erläutert.

Diese zeigt eine Gelenkkugel 1 für ein künstliches Hüftgelenk. Diese Gelenkkugel 1 wird mit einem femoralen Hüftstiel (nicht dargestellt) verbunden. Die Gelenkkugel 1 arbeitet im implantierten Zustand mit einer künstlichen Gelenkpfanne (nicht dargestellt) zusammen.

In die Kugeloberfläche eingelassen sind im abgebildeten Ausführungsbeispiel drei Nuten 2, die einen gerundeten Querschnitt aufweisen, wie an den Rändern der Abbildung zu erkennen ist.

Die Nuten 2 verlaufen zirkulär um die Polachse P herum.

Wenigstens drei solcher Nuten 2 sind in die Kugeloberfläche eingelassen. Die Anzahl kann jedoch variieren bis maximal zehn, wobei die Breite der Nuten dann entsprechend abnimmt.

## Patentansprüche

1. Gelenkkugel (1) für ein künstliches Hüftgelenk, die dazu geeignet ist, in einer künstlichen Hüftgelenkspfanne eine Rotations- und Schwenkbewegung auszuführen,
**gekennzeichnet durch**
wenigstens drei zirkulär um die Polachse (P) verlaufende Nuten (2), die ohne Verbindungskanäle untereinander in die Kugeloberfläche eingebracht sind.

2. Gelenkkugel nach Anspruch 1, **dadurch gekennzeichnet, dass** die Nuten (2) eine Tiefe von bis zu 2 mm aufweisen.

3. Gelenkkugel nach Anspruch 2, **dadurch gekennzeichnet, dass** die Nuten (2) eine Tiefe von 1 mm aufweisen.

4. Gelenkkugel nach einem der Ansprüche 1 bis 3, **dadurch gekennzeichnet, dass** die Nuten (2) einen gerundeten Querschnitt aufweisen.
